Europäisches Patentamt

**European Patent Office**

**Office européen des brevets**

(19)

(11) Numéro de publication: **0 040 138**
**A1**

# (12) DEMANDE DE BREVET EUROPEEN

(21) Numéro de dépôt: **81400708.4**

(22) Date de dépôt: **05.05.81**

(51) Int. Cl.³: **A 61 B 17/38**

(30) Priorité: **08.05.80 FR 8010330**

(43) Date de publication de la demande:
**18.11.81 Bulletin 81/46**

(84) Etats contractants désignés:
**AT BE CH DE GB LI NL SE**

(71) Demandeur: **LABORATOIRES BIOTROL S.A.**
**1, rue du Foin**
**F-75140 Paris Cedex 03(FR)**

(72) Inventeur: **Piton, Jacques**
**239, rue de Pessac**
**F-33000 Bordeaux(FR)**

(74) Mandataire: **Phélip, Bruno et al,**
**c/o Cabinet HARLé & LECHOPIEZ 21, rue de La**
**Rochefoucauld**
**F-75009 Paris(FR)**

(54) **Sonde bipolaire pour électrocoagulation.**

(57) Sonde bipolaire pour électrocoagulation en courant électrique continu.

Elle comprend une anode et une cathode aptes à être introduites coaxialement dans un cathéter d'angiographie classique, l'anode étant située à la partie distale de la sonde et la cathode étant disposée en amont et à une certaine distance de l'anode sur la sonde, ainsi qu'une gaine en matière souple dans laquelle sont noyés les conducteurs reliant les électrodes à la source de courant.

Application notamment à des dévascularisations localisées.

EP 0 040 138 A1

Croydon Printing Company Ltd

## Sonde bipolaire pour électrocoagulation

La présente invention concerne une sonde bipolaire pour électrocoagulation, et plus particulièrement une sonde bipolaire /d'électrocoagulation par voie endoartérielle utilisant un courant électrique continu.

On connaît déjà divers dispositifs destinés à réaliser une électrocoagulation, qui est une technique de destruction des tissus et qui permet notamment de traiter certaines tumeurs sans risque de dissémination ni d'hémorragie. Par exemple les demandes de brevets français publiées sous les numéros 2.355.521 et 2.355.520 décrivent respectivement une pince pour électrocoagulation et des appareils électrochirurgicaux à transistors produisant sélectivement des courants de haute fréquence, haute tension, d'incision et de coagulation. Ces dispositifs et appareils ne donnent cependant pas satisfaction dans tous les cas.

On connaît aussi (voir la DE-OS 2.324.658) un dispositif d'électrocoagulation sous courant alternatif à haute fréquence comprenant, dans une de ses formes de réalisation, deux électrodes annulaires séparées par un manchon isolant, tandis que l'extrémité distale du dispositif a la forme d'une demi-bille et est faite de matière isolante. D'autre part la DE-OS 2.513.868 concerne un dispositif pour le traitement des tissus malades fonctionnant sous courant alternatif de haute fréquence et comprenant des électrodes distales qui peuvent être agencées pour pincer ou enserrer le tissu à cautériser.

On connaît également un dispositif d'embolisation par courant électrique continu comprenant une électrode positive introduite à proximité immédiate de l'endroit où on veut réaliser un caillot et une électrode négative placée en un point approprié sur la peau du patient. A la suite des travaux d'Abramson

/Abramson (H.A.), Am. J.Med. Sci.,1924,167, pp.702-710_7 et de ceux de Phillips, Salazar et Thompson /Phillips (J.F.), Invest. Radiol., 1973,8,pp.295-304 ; Phillips (J.F.) et al., Radiology, 1975,115,pp.319-321 ; Salazar (A.E.), Cir. Res.,1961,9, pp-1351-1356 ; et Thompson (W.M.) et al.,Invest. Radiol.,1977, 12 pp.146-153_7, on a pu en effet montrer l'existence d'une polarisation des parois artérielles et des éléments figurés du sang /Piton (J.) et al., J.Neuroradiol.,1978,5. pp.139-152 et Neuroradiol.,1978, 16, pp.385-388_7 et établir que la dépolarisation artificiellement créée par une électrode positive entraînait une lésion de l'intima et la formation d'un caillot adhérent à la paroi artérielle. L'électrocoagulation par électrode positive en courant continu a permis des interventions parfaitement sélectives, sans présenter les risques inhérents aux autres techniques d'embolisation, à savoir un risque de nécrose avec les embols fluides et, quel que soit le matériel utilisé, un risque de reflux du caillot dans un territoire noble. Une technique d'oblitération artérielle par voie endo-vasculaire a ainsi été développée (Piton (J.), J.Radiol.,1979, 60, n° 12, pp.799-808), utilisant un système relativement simple et permettant d'obtenir des résultats durables au niveau des artères électrocoagulées.

Toutefois, un tel système faisant intervenir une électrode négative externe entraîne souvent des complications au niveau de celle-ci, sous la forme de petites brûlures cutanées en regard des points d'appui de cette électrode. Il a cependant été possible de supprimer cet inconvénient en utilisant une électrode négative en tissu imbibé d'électrolyte.

Mais surtout cette technique convient seulement pour le traitement des petites artères n'ayant pas un diamètre de plus de 5 mm, et pas pour les artères de plus gros débit comme par exemple les artères rénales ou les artères hépatiques. Pour ces grosses artères, de même d'ailleurs que pour les fistules artério-veineuses à gros débit, on ne peut en effet obtenir une thrombose complète qu'après des temps de fonctionnement très longs de l'électrode active, et il avait jusqu'ici uniquement été proposé, pour tenter de remédier à cette situation, soit d'augmenter la surface de contact de cette électrode, soit de procéder au préalable à une obstruction artérielle temporaire par sonde à ballonet double voie en amont du point où doit être effectuée l'électrocoagulation.

On a maintenant trouvé qu'on peut réaliser en des temps notablement réduits, ainsi qu'avec des résultats optimaux et de moindres effets secondaires, l'embolisation par / électrocoagulation au moyen de courant électrique continu, aussi bien des petites que des grosses artères et même des fistules artério-veineuses à gros débit, grâce à une sonde bipolaire originale introduite coaxialement dans un cathéter d'angiographie classique.

La présente invention a donc fondamentalement pour objet une sonde bipolaire pour électrocoagulation en courant électrique continu, reliée en fonctionnement à un générateur de courant continu ou à un redresseur alimenté par une source de courant alternatif, caractérisée en ce qu'elle comprend une anode et une cathode aptes à être introduites coaxialement dans un cathéter d'angiographie classique, l'anode étant située à la partie distale de la sonde et la cathode étant disposée en amont et à une certaine distance de l'anode sur la sonde, ainsi qu'une gaine en matière souple dans laquelle sont noyés les conducteurs reliant les électrodes à la source de courant.

Dans la pratique, les électrodes peuvent être réalisées en l'un quelconque des métaux ou alliages électroconducteurs connus, et notamment le platine, l'argent, un acier inoxydable, le cuivre ou le cuivre étamé, avec une préférence marquée pour l'acier inoxydable. Les dimensions et formes de ces électrodes peuvent être quelconques, pour autant qu'elles demeurent compatibles avec les caractéristiques dimensionnelles du cathéter vecteur utilisé ; leur choix dépend en outre de la vitesse et de la taille de l'embolisation recherchée, et il incombe à l'homme de l'art, éventuellement après des essais de routine, d'apporter aux électrodes de la sonde selon l'invention les dimensions et formes qui conviennent le mieux au but à atteindre, dans plusieurs cas types ou dans chaque cas d'espèce. A titre indicatif, on peut préconiser une anode constituée par un fil spiralé, ou une plaque enroulée, autour de l'axe de la sonde sur une longueur de 5-20 mm environ, et une cathode constituée d'un manchon d'environ 3-20 mm de long, entourant la sonde en amont de l'anode et laissant subsister entre elle et cette anode un espace de 5-60 mm environ, qui peut être maintenu à nu ou de préférence être lui-même noyé dans la même matière souple que celle qui gaîne les conducteurs portant ces électrodes sur leurs parties libres respectives.

L'essentiel est, du point de vue électrique, que les matériaux mis en oeuvre pour les éléments conducteurs assurent une résistance de ceux-ci à des courants dont l'intensité peut varier de 8 à 12 mA environ, pour une tension continue de 6 à 10 V environ, cette résistance et cette tension correspondant aux conditions de mise en oeuvre de la sonde selon l'invention. Le générateur de courant continu ou le redresseur de courant alternatif du réseau doit être conçu de telle manière qu'on puisse faire varier indifféremment l'ampérage et/ou la tension, dans les plages de valeurs susdites.

La matière de gainage peut être, par exemple, choisie parmi du polyéthylène, ou toute autre matière plastique souple non électroconductrice et autorisée par la Pharmacopée française, telle que du PTFE, les polyuréthanes, les polyamides, entre autres ou des silicones et dérivés.

De manière générale, les mensurations de la sonde et de ses divers composants peuvent être variables et sont fonction du calibre et de la longueur des artères à emboliser ; l'ensemble doit pouvoir se coulisser dans un cathéter d'angiographie de toute taille, comme par exemple de calibre 7F pour les grosses artères et de calibre 5F pour les petites artères, sans que cela soit limitatif.

Cette sonde est bien sûr radio-opaque. Elle doit être suffisamment souple pour assurer le cathétérisme atraumatique /des artères à emboliser. Cette souplesse est d'autant meilleure que la partie constituant l'anode est réalisée en un fil spiralé et est d'une longueur suffisante pour pouvoir acquérir un rayon de courbure approprié au cathétérisme sélectif du vaisseau à emboliser.

Selon une variante particulièrement intéressante, la sonde selon l'invention peut encore comporter, de préférence, noyé dans l'axe de la gaine susdite, un système à fibres optiques apte à conduire un rayon laser provenant d'une source appropriée et débouchant sensiblement dans l'axe du volume creux défini par l'anode à l'extrémité distale de la sonde.

Pour disposer d'un kit d'embolisation complet, il convient en outre d'ajouter à cette sonde un cathéter d'angiographie où elle doit pouvoir coulisser librement, et éventuellement le dispositif approprié pour produire du courant électrique continu.

Pour la mise en oeuvre pratique du système d'électrocoagulation selon l'invention, on peut faire suivre le cathétérisme initial d'une ou plusieurs angiographie(s) de contrôle, si on le souhaite, et mettre la sonde selon l'invention en place aussi près que possible du point de l'artère où on désire effectuer l'électrocoagulation. Selon les cas de maladies à traiter, on peut retirer le cathéter d'angiographie en fin d'opération ou le laisser en place.

La facilité et la rapidité de l'obstruction vasculaire réalisée avec la sonde conforme à l'invention sont essentiellement fonction, d'une part du débit circulatoire et du calibre artériel, d'autre part de la surface d'anode en contact avec le sang et la paroi artérielle.

En fonctionnement, il peut se produire une électrolyse partielle ou totale de l'anode, entraînant une corrosion plus ou moins complète de celle-ci et même parfois une libération de son fragment distal dans l'artère embolisée, où ce fragment se trouve retenu dans le caillot et consolide la thrombose.

Dans tous les cas les lésions créées sont définitives et, les interventions étant parfaitement sélectives, autorisent l'utilisation du système selon l'invention pour des dévascularisations localisées, notamment en vue d'opérations chirurgicales d'ablation.

La sonde endo-vasculaire selon l'invention agit sur les protéines circulantes et sur les ions contenus dans le sang, en les transformant par électrolyse ; elle a pour effet principal de modifier la structure tertiaire de la fibrine, en la rendant non lysable.

REVENDICATIONS

1. Sonde bipolaire pour électrocoagulation en
courant électrique continu, reliée en fonctionnement
à un générateur de courant continu ou à un redresseur
alimenté par une source de courant alternatif, caractérisée en ce qu'elle comprend une anode et une
cathode aptes à être introduites coaxialement dans
un cathéter d'angiographie classique, l'anode étant
située à la partie distale de la sonde et la cathode
étant disposée en amont et à une certaine distance
de l'anode sur la sonde, ainsi qu'une gaine en
matière souple dans laquelle sont noyés les conducteurs reliant les électrodes à la source de courant.

2. Sonde bipolaire selon la revendication 1, caractérisée en ce que les électrodes sont réalisées en
acier inoxydable.

3. Sonde bipolaire selon l'une des revendications
1 ou 2, caractérisée en ce que l'anode est formée
d'un fil spiralé, ou d'une plaque enroulée, autour
de l'axe de la sonde sur une longueur de 5-20 mm
environ, et la cathode est constituée d'un manchon
d'environ 3-20 mm de long, entourant la sonde en
amont de l'anode et laissant subsister entre elle
et cette anode un espace de 5-60 mm environ.

4. Sonde bipolaire selon l'une quelconque des
revendications 1 à 3, caractérisée en ce que la
matière souple de gainage est du polyéthylène, du
PTFE, un polyuréthane, un polyamide, ou une silicone
ou un dérivé de celle-ci.

5. Sonde bipolaire selon l'une quelconque des
revendications 1 à 4, caractérisée en ce qu'elle
comporte, de préférence noyé dans l'axe de la gaine
en matière souple, un système à fibres optiques apte
à conduire un rayon laser provenant d'une source
appropriée et débouchant sensiblement dans l'axe du
volume creux défini par l'anode à l'extrémité distale
de la sonde.

Office européen
des brevets

**RAPPORT DE RECHERCHE EUROPEENNE**

EP 81 40 0708

| Catégorie | Citation du document avec indication, en cas de besoin, des parties pertinentes | Revendica-tion concernée |
|---|---|---|
| | **DOCUMENTS CONSIDERES COMME PERTINENTS** | |
| D | DE - A - 2 324 658 (WOLF)<br>* Figures; page 5, ligne 6 - page 6, ligne 10 *<br><br>-- | 1,3 |
| D | DE - A - 2 513 868 (OLYMPUS OP-TICAL)<br>* Page 5, lignes 9-29; figures *<br><br>-- | 1,3 |
| A | DE - A - 2 521 719 (DELMA)<br>* Revendications 1-5; figures *<br><br>---- | 5 |

**CLASSEMENT DE LA DEMANDE (Int. Cl.³)**

A 61 B 17/38

**DOMAINES TECHNIQUES RECHERCHES (Int. Cl.³)**

A 61 B

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 04-08-1981 | STEENBAKKER |

OEB Form 1503.1  06.78